# EUROPÄISCHE PATENTSCHRIFT

(11) **EP 0 334 306 B1**
(45) Veröffentlichungstag und Bekanntmachung des Hinweises auf die Patenterteilung: **11.09.1996**
(21) Anmeldenummer: 89105078.3
(22) Anmeldetag: 21.03.1989
(51) Int. Cl.: A61M 35/00

(54) **Verwendung einer Vorrichtung mit einem Wirkstoffreservoir für gesteuerte Wirkstoffabgabe**
Use of a device having a reservoir containing an active substance for the controlled release of the active substance
Utilisation d'un dispositif comprenant un réservoir pour une substance active pour la libération commandée de la substance active.

(30) Priorität: 24.03.1988 DE 3809978
(43) Veröffentlichungstag der Anmeldung: 27.09.1989
(73) Patentinhaber: LTS LOHMANN Therapie-Systeme GmbH, 56567 Neuwied (DE)
(72) Erfinder: Herrmann, Fritz Dr., D-5450 Neuwied 12 (DE)
(74) Vertreter: Neidl-Stippler, Cornelia, Dr.

(56) Entgegenhaltungen:
- EP-A- 0 242 827
- EP-A- 0 259 113
- EP-A- 0 259 219
- WO-A-87/00042

## Beschreibung

Die Erfindung betrifft die Verwendung einer Vorrichtung zur Abgabe von Wirkstoffen an einen Akzeptor.

Die Erfindung betrifft also allgemein die Verwendung von Vorrichtungen mit Reservoiren mit Abgabeflächen zur gesteuerten, über die Zeit der Anwendung abnehmenden Abgabe von Wirkstoffen aus dem Reservoir an feste, flüssige oder gasförmige Akzeptoren.

Unter Wirkstoffen werden in diesem Zusammenhang Stoffe verstanden, die eine erwünschte Wirkung - sei es eine optische, eine physikalische oder eine chemisch/biologische auf einem Gebiet der Technik, der Medizin oder der Biologie, eingeschlossen der Schädlingsbekämpfung, ausüben.

Die Abgabe von Wirkstoffen aus dem Reservoir an ein bestimmtes Akzeptormedium kann bezüglich des zeitlichen Verlaufs im Prinzip auf zwei Arten erfolgen.

Einmal kann die gesamte gewünschte Menge an Wirkstoff in einer einzigen Portion zugeführt werden und zum anderen kann die gewünschte Gesamtmenge aufgeteilt in mehr oder weniger großen Teilmengen diskontinuierlich oder kontinuierlich über einen bestimmten Zeitraum verteilt an das Akzeptormedium abgegeben werden. Mit dem letzteren Fall befaßt sich die vorliegende Erfindung.

Es handelt sich hier um die Abgabe von Wirkstoff aus einem Reservoir an ein bestimmtes Akzeptormedium. Neben der Kontrollierbarkeit der über die Zeit und in der Zeiteinheit von dem Reservoir abgegebenen Wirkstoffmenge wird hier auch eine Steuerbarkeit der Wirkstoffabgabechrakteristik gefordert. Diese Forderung ist in vielen Fällen unabdingbar und spielt beispielsweise bei den sog. transdermalen therapeutischen Systemen eine wichtige Rolle.

Dies sind wirkstoffenthaltende Vorrichtungen bzw. Darreichungsformen, die einen Wirkstoff oder mehrere in vorbestimmter Rate kontinuierlich über einen festgelegten Zeitraum an die Haut abgeben. Es sind viele Anstrengungen unternommen worden, diese Systeme zu verwirklichen.

Das Ziel, eine über einen gewünschten Zeitraum annähernd gleichbleibende Wirkstoffabgabe einzustellen, wurde erreicht. Für andere Wirkstoffabgabecharakteristika liegen Lösungsvorschläge vor, die jedoch noch nicht voll befriedigen. So ist man bei einer gesteuerten Abnahme der Wirkstoffabgabe über die Applikationszeit (bei Arzneimitteln bezeichnet man dieses bspw. als Ausschleichen - dieses wird bspw. bei cortisonhaltigen Präparaten oder auch bei Nitroglyceringabe angestrebt - es kann auch wegen einer Anpassung an den Biorhythmus des zu behandelnden Organismus notwendig sein.) auf die stoffliche Zusammensetzung der Reservoirmatrix angewiesen. Dies bietet allerdings nur einen begrenzten Spielraum, da auch die Grundvoraussetzung der Diffusionsfähigkeit des Wirkstoffes in der Matrix erfüllt sein muß. Daneben bieten die Konzentration und/oder die Konzentrationsverteilung des Wirkstoffes in der Matrix Möglichkeiten der Steuerung im gewünschten Sinne, was allerdings nur in Sonderfällen genutzt werden kann.

In der US-A 4 564 364 wird der Vorschlag gemacht, das Wirkstoffreservoir in Volumenbereiche zu unterteilen, in denen die Wirkstoffkonzentration zum Teil unterhalb und zum Teil oberhalb der Sättigungskonzentration eingestellt ist. Durch gezielte geometrische Ausgestaltung der Volumenbereiche kann Einfluß auf die Wirkstoffabgabecharakteristik genommen werden. Abgesehen davon, daß die Herstellung derartiger Systeme kompliziert und aufwendig ist, kann der hier angegebenen Lehre zum technischen Handeln die Lösung des Problems einer gezielten gesteuerten Abnahme der Wirkstoffabgabe über die Applikationszeit des Systems nicht entnommen werden.

Ein anderer Weg zur Steuerung der Wirkstoffabgabe aus Matrixreservoiren wird in der DE-B 33 15 272 beschritten. Hier ist das Reservoir aus mindestens zwei zur Abgabefläche parallelen Schichten aufgebaut, die Wirkstoffkonzentrationen oberhalb der Sättigungskonzentration enthalten, wobei die Konzentration mit steigender Entfernung der Schicht von der Abgabefläche zunimmt. Es gelingt damit zwar, die Abgaberate für diese gewünschte Zeit auf einem bestimmten Niveau zu halten, doch auch hier kann eine gezielte Steuerung der Abnahme der Wirkstoffabgaberate nicht erreicht werden. Die EP-A O 227 252 schlägt Reservoirs vor, deren Kinetik alleine durch die Diffusionsgeschwindigkiet des Wirkstoffes bzw. die Erschöpfungskinetik des Wirkstoffes im Matrixmaterial bestimmt wird.

Aus der WO 87/00042 ist eine transdermale Vorrichtung zur Abgabe von VERAPAMIL bekannt geworden, mittels derer das VERAPAMIL durch eine permeable Matrix aus Silikonelastomer an die Haut abgegeben wird. Diese Vorrichtung verwirklicht eine gleichmäßige Abgabegeschwindigkeit.

Aus der DE-U-8709810.5 sind medizinische Pflaster bekannt, die halbkugelförmige Wirkstoffreservoirs aufweisen, die jedoch nur eine gleichmäßige Wirkstoffabgabe liefen.

Es werden dort über diese die Abgabegeschwindigkeit beeinflussenden materialspezifischen Größen keinerlei Anregungen gegeben, wie die dort beschriebenen transdermalen Systeme so abgewandelt werden können, daß sie für eine kompliziertere Wirkstoffverabreichung, wie sie insbesondere bei sogenannter "ausschleichender Gabe vom Wirkstoffen" erwünscht, ist, geeignet sind.

Es ist daher Aufgabe der Erfindung, eine Reservoir zu schaffen, das eine definiert gesteuerte Abnahme der Wirkstoffabgabe über eine gewünschte oder notwendige Zeit erermöglicht.

Überraschenderweise wurde nun die Lösung des Problems darin gefunden, daß durch die Verwendung einer Vorrichtung zur Abgabe von Wirkstoffen an einen Akzeptor zur über die Zeit der Anwendung gesteuerten Abnahme von Wirkstoffen mit mindestens einem Wirkstoffreservoir mit einer Abgabefläche, die mit dem Akzeptor in Kontakt steht, wobei mindestens eine zur Abgabefläche paralelle Querschnittsfläche des Reservoirs kleiner ist als die Abgabefläche und keine zur Abgabefläche paralelle Querschnittsfläche des Reservoirs größer ist als die Abgabefläche und durch die geometrische Form des Reservoirs die Abgabecharaktaristik bestimmt ist, nun eine definiert gesteuerte Abnahme der Wirkstoffabgabe über die Zeit sichergestellt ist.

Vorteilhafte Weiterbildungen der Erfindung ergeben sich aus den Unteransprüchen.

Die Abgabemenge eines Stoffes über die Zeit aus einem Reservoir durch die Abgabefläche ist direkt proportional der Größe dieser Abgabefläche. Das Reservoir selbst kann dabei aus reinem Wirkstoff oder aus einer über die Zeit der Anwendung unverändert bleibenden Reservoirmasse bestehen, in der Wirkstoff verteilt ist. Im ersten Fall führt die Abgabe von Wirkstoff zu einer Abtragung des Reservoirs unter Ausbildung immer neuer Abgabeflächen. Ist der Querschnitt des Reservoirs parallel zur Abgabefläche an mindestens einer Stelle verkleinert, bildet sich dort eine in der Größe verringerte Abgabefläche aus, so daß sich damit eine Abnahme der Wirkstoffabgabe einstellt.

Im zweiten Fall - Reservoirmasse mit darin verteiltem Wirkstoff - liegen die Verhältnisse anders. Die bei der Herstellung des Reservoirs gebildete Abgabefläche bleibt in ihrer ganzen Größe über die gesamte Applikationszeit erhalten. Der durch sie austretende Wirkstoff hinterläßt im Reservoir eine zur Abgabefläche parallele Zone mit gegenüber dem übrigen Reservoir verminderter Wirkstoffkonzentration, deren weitere Ausdünnung durch Nachlieferung von Wirkstoff aus dem höherkonzentrierten Teil des Reservoirs vermieden wird. Die Abgabemenge durch die Abgabefläche wird damit von dem Ausmaß des nachgelieferten Wirkstoffes bestimmt, wodurch die Dicke der Zone mit verringerter Konzentration ständig zunimmt. Es bildet sich demnach parallel zur Abgabefläche eine Art Trennflächezwischen den Reservoirteilen mit verringerter Konzentration und höherer Ursprungskonzentration, deren Abstand zur Abgabefläche während der Applikation ständig ansteigt und deren Größe der des Querschnittes des Reservoirs parallel zur Abgabefläche entspricht. Die Größe dieser Trennfläche und damit der Querschnitt des Reservoirs bestimmt aber die Abgaberate von Wirkstoff durch die Abgabefläche. Gemäß der Erfindung wird nun die Größe der Trennfläche durch eine entsprechende geometrische Ausgestaltung des Reservoirs definiert und gezielt verkleinert, so daß die Abgabemenge an Wirkstoff aus dem Reservoir gegenüber der Initialmenge definiert und gesteuert reduzierbar ist.

Die Querschnittsverkleinerung kann über das gesamte Reservoir kontinuierlich sein, wobei ein linearer oder auch nicht linearer Verlauf der Verkleinerung unterschieden werden kann. Auch eine diskontinuierliche, wie z.B. stufenartige Querschnittsverkleinerung kann im Bedarfsfall realisiert werden. Der Verlauf kann auch hier linear oder nicht linear sein.

Als besonders geeignete geometrische Formen für das Reservoir haben sich beispielsweise Kegel und Kegelstümpfe, Pyramiden und Pyramidenstümpfe, Tetraeder und Tetraederstümpfe sowie Halbkugeln, Kugelsegmente und Kugelschichten bewährt. Natürlich kann das Reservoir auch so gestaltet werden, daß die Querschnittsverkleinerung nur in einem Teil des Reservoirs ausgebildet ist. Symmetrieelemente im Reservoir sind nicht unabdingbare Voraussetzungen für erfindungsgemäße Problemlösungen. Bei der Größendimensionierung des Reservoirs kann die Abgabefläche in einer Richtung eine maximale Ausdehnung aufweisen, die bis zum 1OO-fachen der Ausdehnung in der dazu senkrechten Richtung beträgt. Im Spezialfall einer rechteckigen Abgabefläche entspräche dies einer 100-fach größeren Länge als Breite.

Damit nur eine bestimmte Außenfläche des Reservoirs die Funktion der Wirkstoffabgabe übernimmt, wird auf allen anderen Flächen mindestens eine Schicht fixiert, die für die Bestandteile des Reservoirs undurchlässig ist. Das Reservoir kann auch zumindest teilweise in eine entsprechende Vertiefung in einem Trägermaterial eingelassen sein, wobei es mit mindestens einer Fläche, ausgenommen die Abgabefläche, in der Vertiefung fixiert sein kann. Um den Beginn der Wirkstoffabgabe genau terminieren zu können und um die Abgabefläche vor Beschädigungen zu schützen, ist die Abgabefläche bevorzugt durch ein mindestens einschichtiges, wieder ablösbares und für die Bestandteile des Reservoirs undurchlässiges Flächengebilde geschützt.

Mindestens ein derartiges Reservoir kann Bestandteil einer erfindungsgemäß verwendeten Vorrichtung zur gesteuerten über die Zeit der Anwendung abnehmenden Abgabe von Wirkstoffen sein.

In bestimmten Fällen kann die Verwendung der Kombination mehrerer Reservoire in einer derartigen Vorrichtung bevorzugt sein, die sich in ihrer geometrischen Ausgestaltung auch voneinander unterscheiden können. Vorzugsweise umfassen die Vorrichtungen Elemente, die eine Fixierung der Vorrichtung am Applikationsort ermöglichen. Besonders bewährt haben sich dazu haftklebende Bereiche der Vorrichtung. Das Reservoir umfaßt mindestens einen Wirkstoff, der an feste, flüssige oder gasförmige Akzeptormedien abgegeben werden kann, wobei der/die Wirkstoff(e) bevorzugt im Bereich der Technik, der Human- und Veterinärmedizin, der Kosmetik und der Schädlingsbekämpfung seine/ihre Wirkung entfaltet(n). Bevorzugt sind Wirkstoffe für die Humanmedizin in Vorrichtungen, die als transdermale therapeutische Systeme zu bezeichnen sind.

Für die Herstellung einer erfindungsgemäß verwendeten Vorrichtung wird das Reservoir vorzugsweise in einer entsprechenden Form gebildet. Die dazu eingesetzten Verfahren sind vorzugsweise Abkühlen einer Schmelze der Reservoirmasse, Abdunsten von Löse- oder Dispergiermittel, Kalt- oder Heißpressen von Reservoirmasse und Profil-Extrusion oder auch die Vernetzung von Polymeren durch Strahlung oder Wärme. Die Ummantelung des Reservoirs außerhalb der Abgabefläche - kann dann vorzugsweise durch Laminieren, Besprühen und Tauchen erfolgen.

Weitere bevorzugte Verfahren zur Herstellung von erfindungsgemäß eingesetzten Vorrichtungen, bei denen zumindest ein Teil des Reservoirs von geeigneten Vertiefungen in einem Trägermaterial aufgenommen wird, bestehen darin, daß die Vertiefungen durch Tiefziehen oder Prägen oder schon bei der Formgebung des Trägermaterials erzeugt werden.

Die Vorrichtungen zur erfindungsgemäßen Verwendung werden vorzugsweise in der Technik, der Human- und Veterinärmedizin, der Kosmetik und der Schädlingsbekämpfung verwendet.

Eine besonders bevorzugte erfindungsgemäße Verwendung der Vorrichtungen ist die als transdermale therapeutische Systeme.

Die vorliegende Erfindung bietet weitgehende Möglichkeiten in allen Fällen, wo ein Wirkstoff aus einem Reservoir an irgendein Akzeptormedium so abgegeben werden soll, daß eine gezielte und gesteuerte Abnahme der Abgaberate über die Applikationszeit erreicht wird. Der Kontakt des Reservoirs mit dem Akzeptormedium erfolgt über die Abgabefläche. Diese Abgabefläche ist ein Teil der Oberfläche des dreidimensionalen Reservoirs. Ist das Akzeptormedium ein fester Stoff, so muß die Abgabefläche den Konturen der Kontaktfläche des festen Stoffes angepaßt sein, damit ein kontrollierbarer Übergang des Wirkstoffes gewährleistet, ist. Aus der Sicht der Herstellung werden planare Abgabeflächen bevorzugt. Bei flüssigen und gasförmigen Akzeptormedien wird die Ausgestaltung der Abgabefläche primär von den Herstellungsmöglichkeiten bestimmt, da sich diese Akzeptormedien jeder Form der Abgabefläche anpassen.

Die erfindungsgemäß verwendete Vorrichtung umfaßt mindestens eines der beschriebenen Reservoire. In bestimmten Fällen jedoch sind auch Vorrichtungen mit mehreren Reservoiren sinvoll. So kann es sein, daß bei einer bestimmten geforderten Abgabecharakteristik und Abgabemenge das dazu benötigte Reservoir in seiner Dimensionierung zu unhandlich wird. Hier wird daher die Abgabefläche in Teilflächen zerlegt, die dann ihrerseits die Abgabeflächen von mehreren kleineren Reservoiren bilden. Diese können in einer Vorrichtung zusammengefaßt werden. Die Möglichkeiten der geometrischen Anordung dieser kleiner Reservoire zueinander werden nicht durch die angestrebte Abgabecharakteristik der Wirkstoffe beschränkt.

In anderen Fällen sind mehrere Reservoire in einer Vorrichtung angezeigt, wenn es darum geht, mit einer Vorrichtung zwei miteinander unverträgliche Wirkstoffe gleichzeitig nach derselben oder auch unterschiedlichen Abgabecharakteristik zu applizieren, d.h. es sind auch Reservoire unterschiedlicher geometrischer Ausgestaltung miteinander kombinierbar.

Im DE-U 87 09 810.5 ist ein medizinisches Pflaster beschrieben, das im Trägermaterial geometrisch definierte Mulden besitzt, die vor der Applikation mit Reservoirmasse, wie z.B. Salbe, gefüllt werden. Die geometrisch definierte Ausbildung der Mulden soll das Volumen der zur Applikatioon eingesetzten Reservoirmasse möglichst genauer definieren, als es mit den bis dahin gebräuchlichen Vorrichtungen möglich war. Eine gesteuerte, definierte Abgabecharakteristik bezüglich der Abnahme der Wirstoffabgabe über die Applikationszeit ist in dieser Druckschrift nicht zu erkennen.

Neben der gemäß der Erfindung gewählten geometrischen Grundform des Reservoirs kann die Abgabecharakteristik weiterhin durch Festlegung frei wählbarer Strukturelemente der Grundform beeinflußt werden. So hängt zum Beispiel die Abgabecharakteristik eines kegelförmigen Reservoirs vom frei wählbaren Winkel in der Kegelspitze ab. Bei kleinen Winkel resultiert ein relativ hoher Kegel, der eine ziemlich flache Abnahme der Wirkstoffabgabe besitzt, während bei großen Winkeln ein niedrigerer Kegel resultiert mit einem steilen Abfall der Wirkstoffabgaberate. Damit wird dokumentiert, wie vielfältig die Variationsmöglichkeiten in der Reservoirgestaltung zum Einsatz gemäß der Erfindung überhaupt sind.

Die Abgaberate, d.h. die Abgabemenge des Wirkstoffes pro Zeiteinheit, wird noch von anderen Faktoren bestimmt. Bei Reservoiren, die aus reinem Wirkstoff oder Wirkstoffzubereitungen bestehen und die im Kontakt mit dem Akzeptormedium abgetragen werden, wird die Abgaberate in erster Linie von der Löslichkeit des Wirkstoffes oder der Wirkstoffzubereitung im Akzeptormedium beeinflußt. Als zweiter Faktor ist natürlich die Größe der Abgabefläche zu nennen, die auch bei den über die Applikationszeit volumenkonstanten Reservoiren eine wichtige Rolle spielt. Hier sind noch als weitere Parameter die Löslichkeit des Wirkstoffes in der Reservoirmatrix, die Konzentration des Wirkstoffes in der Matrix, die Konzentrationsverteilung in der Matrix und die Diffusionsfähigkeit des Wirkstoffes in der Marix anzuführen. Nicht zuletzt muß die Temperatur während der Wirkstoffabgabe erwähnt werden, die es im gegebenen Fall ratsam erscheinen läßt, das Reservoir zu kühlen oder auch zu erwärmen.

Die Wahl der geeigneten Reservoirausgestaltung hängt vom Einzelfall ab und kann nicht global definiert werden. Neben der gewünschten Abgabecharakteristik spielen dabei alle oben angeführten Parameter eine Rolle.

Aus dem Reservoir wird dann eine Abgabevorrichtung, wenn alle nicht zur Abgabe bestimmten Flächen keinen direkten Kontakt zum Akzeptormedium haben. Sie können durch sowohl für die Bestandteile des Reservoirs als auch für das Akzeptormedium undurchlässige Flächengebilde abgedeckt sein.

Eine andere Möglichkeit ist vorzugsweise im Einbetten des Reservoirs in Vertiefungen eines Trägermaterials zu sehen, das dieselben Funktionen wie das oben genannte Flächengebilde ausübt. Die Vorrichtung kann gegebenenfalls am Ort der Applikation fixiert werden. Dazu können äußere Hilfsmittel eingesetzt werden, oder die Vorrichtung selbst ist mit Fixierelementen versehen. Im letzten Falle werden bevorzugt haftklebende Bereiche vorgesehen. So können bei festen Akzeptormedien das Reservoir selbst und damit die Abgabefläche haftklebend sein, oder die Abgabefläche ist mit haftklebenden Bereichen ausgestattet, die einen ungehinderten Durchgang der Wirkstoffe erlauben. Die haftklebenden Bereiche können auch an anderen Flächen der Vorrichtung vorgesehen sein, wenn die Akzeptormedien flüssig oder gasförmig sind.

Natürlich muß die Abgabefläche vor der Applikation der Vorrichtung geschützt sein, was vorzugsweise durch ein Flächengebilde bewerkstelligt wird, das für die Bestandteile des Reservoirs undurchlässig und vor der Applikation wieder ablösbar ist.

Die Stoffe, die zur Herstellung der Vorrichtung einsetzbar sind, richten sich nach deren Anforderungen im gegebenen Fall und sind dem Fachmann bekannt. Auch die mit der Vorrichtung applizierbaren Wirkstoffe sind so vielfältig und ebenfalls dem Fachmann bekannt, daß eine erschöpfenden Aufzählung nicht möglich ist.

Die Figuren sollen die Erfindung weiter erläutern, ohne sie jedoch zu begrenzen. Es zeigen:
- Fig. 1-3:: Längsschnitte erfindungsgemäß verwendeter Reservoire,
- Fig. 4:: Perspektivische Darstellung einer Reservoirausgestaltung,
- Fig 4a:: Perspektivische Darstellung einer Reservoirausgestaltung nach dem Prinzip der Fig. 4,
- Fig. 5:: Perspektivische Darstellung einer weiteren Reservoirausgestaltung,
- Fig. 5a:: Perspektivische Darstellung einer Reservoirausgestaltung nach dem Prinzip der Fig. 5,
- Fig. 6:: Längsschnitt eines Reservoirs mit diskontinuierlicher Querschnittsverkleinerung,
- Fig. 7:: Perspektivische Darstellung eines Reservoirs mit diskontinuierlicher Querschnittsverkleinerung,
- Fig. 8:: Längsschnitt eines weiteren Reservoirs mit diskontinuierlicher Querschnittsverkleinerung,
- Fig. 9:: Schematischer Längsschnitt durch eine erfindungsgemäß verwendete Vorrichtung,
- Fig. 10:: Schematischer Längsschnitt durch eine weitere bevorzugt verwendete Vorrichtung,
- Fig. 11:: Schematischer Längsschnitt durch eine weitere bevorzugt verwendete Vorrichtung mit mehreren Reservoiren,
- Fig. 12:: Schematischer Längsschnitt durch eine verwendete Vorrichtung für zwei verschiedene Wirkstoffe,
- Fig. 13:: Querschnitt entlang der Linie I/I in Fig. 13,
- Fig. 14:: Graphische Darstellung der Querschnittsabnahme bei Kegel und Halbkugel;
- Fig. 15:: Graphische Darstellung der Querschnittsabnahme beim Kegel in Abhängigkeit vom Kegelwinkel, und
- Fig. 16:: Auftragung Flux gegen die Zeit gemäß Beispiel

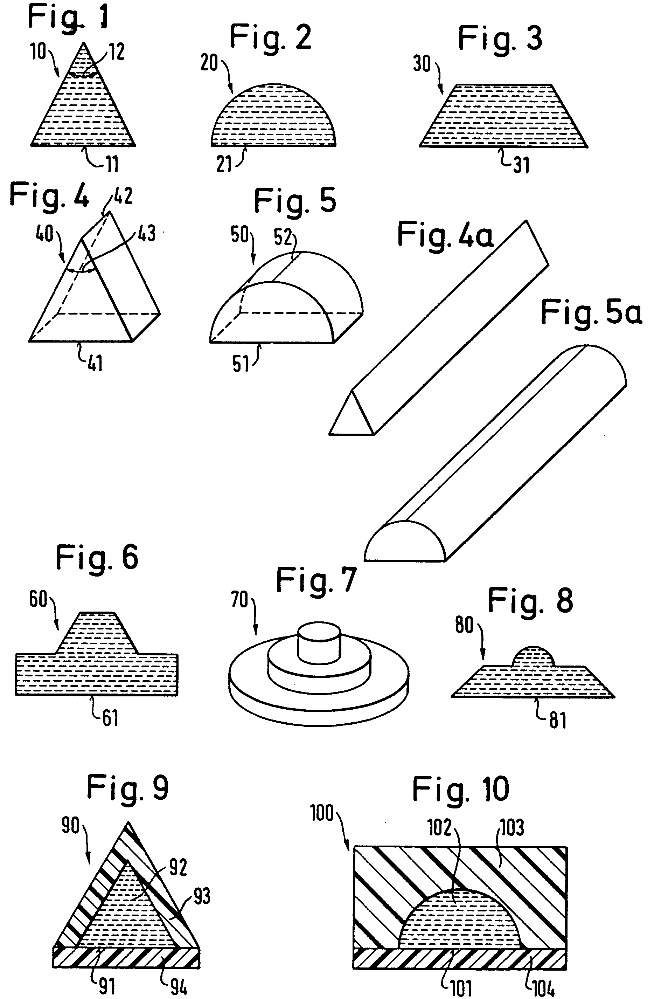

Der dreieckige Längsschnitt 10 in der Fig. 1 eines Reservoirs zeigt mit 11 den Schnitt durch die Abgabefläche. Das dazugehörige Reservoir kann beispielsweise eine Kegel- oder Pyramidenform besitzen. Hier liegt eine kontinuierliche nicht lineare Querschnittsverkleinerung vor. Des weiteren kann 10 ein Schnitt durch die Reservoirform 40 in Fig. 4 oder durch die Form in Fig. 4a sein. Die Querschnittsverkleinerung ist hier kontinuierlich linear. Durch die Wahl des Winkels 12 kann die gewünschte Abgabecharakteristik eingestellt werden. In Fig.2 ist der Längsschnitt 20 eines halbkugelförmigen Reservoirs mit der Abgabefläche 21 dargestellt, wobei der einzige variable geometrische Parameter der Kugeldurchmesser ist.

Daneben gibt 20 aber auch einen Schnitt durch die Form 50 der Fig. 5 und Fig. 5a wieder. Es liegt wieder in allen drei Fällen eine kontinuierliche nichtlineare Querschnittsverkleinerung vor. Diese Reservoirformen sind immer dann angezeigt, wenn die Abgabemenge zum Ende der Applikationszeit steil abfallen soll. Der Längsschnitt 30 in Fig. 3 gehört zu Reservoiren in Form eines Pyramidenstumpfes. Die Abgabefläche ist mit 31 bezeichnet. Die Variationsmöglichkeiten sind die Höhe des Stumpfes und die Größe des Winkels in der Spitze der dazugehörenden Pyramide. Auch hier muß die Querschnittsverkleinerung als kontinuierlich nicht linear bezeichnet werden.

Fig. 4 zeigt die perspektivische Ansicht 40 eines Reservoirs in Zeltform mit 41 als Abgabefläche. Die Variationsmöglichkeit der Länge der oberen Kante 42, wie sie beispielhaft am Reservoir der Fig. 4a dargestellt ist, und des Winkels 43 bestimmen die möglichen Abwandlungen dieser Reservoirform. Die Querschnittsverkleinerung ist hier kontinuierlich linear. Die Abwandlung der Reservoirform 50, die in Fig. 5 perspektivisch wiedergegeben ist, kann nur durch Variation der Abgabefläche 51 und der Länge der Scheitellinie 52 erfolgen. Für letzteren Fall ist in Fig. 5a ein Beispiel gegeben. Die Verkleinerung des Querschnitts ist in diesen Fällen kontinuierlich nicht linear.

Die Ausgestaltung eines Reservoirs mit diskontinuierlicher Querschnittsverkleinerung ist aus dem Längsschnitt 60 in Fig. 6 zu sehen. Die Verkleinerung ist dann linear, wenn das Reservoir an allen Stellen denselben Längsschnitt aufweist. Mit 61 ist die Abgabefläche des Reservoirs der Figur 6 bezeichnet. Der darüberliegende erste Teil des Reservoirs ermöglicht von der Geometrie hier eine weitgehend konstante Wirkstoffabgabe. Nach einer sprunghaften Verkleinerung des Querschnitts ist im zweiten Teil des Reservoirs der Querschnitt kontinuierlich verkleinert.

Auch in Fig. 7 ist ein perspektivisches Bild 70 eines Reservoirs mit diskontinuierlicher Querschnittsverkleinerung dargestellt, wobei der Querschnitt in den einzelnen Segmenten konstant bleibt. Damit ist die Möglichkeit zur stufenweisen Steuerung der Abnahme der Wirkstoffabgabe gegeben. Natürlich sind analoge Reservoirstrukturen auf der Basis vieler anderer geometrischer Formen möglich.

Der Längsschnitt durch ein Reservoir 80 in Fig. 8 zeigt die Kombination eines Kegelstumpfes, der die Abgabefläche 81 bildet, mit einer Halbkugel. Die Querschnittsverkleinerung ist diskontinuierlich und in den einzelnen Teilen nicht linear.

Mit 90 ist in Fig. 9 der Längsschnitt durch eine erfindungsgemäße Vorrichtung zur Steuerung der Wirkstoffabgabe bezeichnet. Das Reservoir 92 entspricht dabei der in Fig. 1 dargestellten Reservoirform 10 und ist an den nicht zur Abgabe des Wirkstoffs bestimmten Flächen mit einer undurchlässigen Schicht 93 versehen. Die Abgabefläche 91 ist durch eine Schutzschicht 94 abgedeckt. Zwischen beiden kann gegebenenfalls noch eine Haftklebeschicht angeordnet sein.

Das Reservoir 102 gemäß Fig. 2 ist bei der Längsschnittzeichnung einer Vorrichtung 100 in Fig. 10 in die Vertiefung in einem Trägermaterial 103 eingelassen. Dabei kann nötigenfalls die Fixierung des Reservoirs in der Vertiefung durch eine Klebeschicht bewerkstelligt werden. Die Schicht 104 schützt die Abgabefläche 101 des Reservoirs 102 vor der Anwendung.

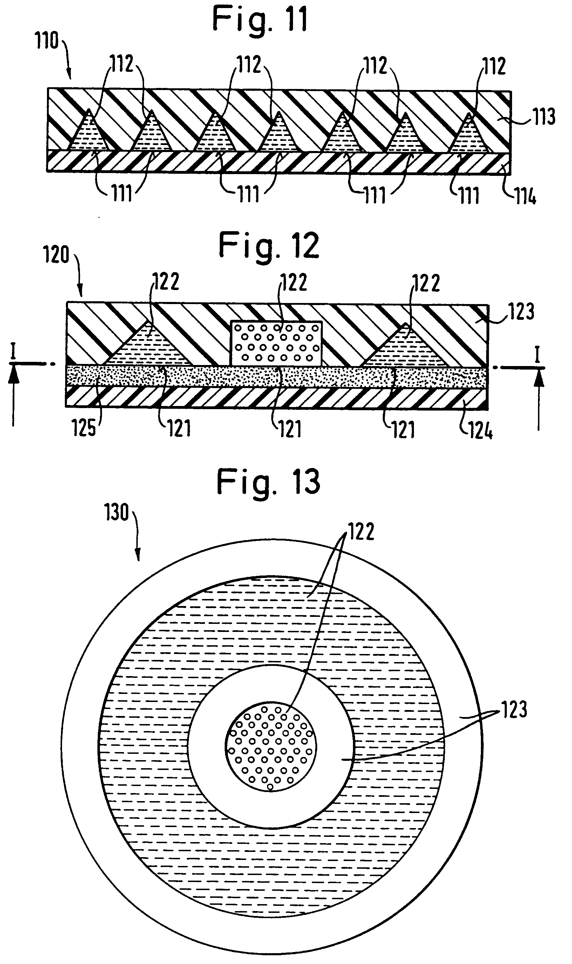

Wird aus irgendeinem Grund die Kombination mehrerer Reservoire in einer Vorrichtung notwendig, so kann Fig. 11 ein beispielhaftes Bild davon geben. Sie zeigt einen Längsschnitt einer Vorrichtung 110, bei der mehrere kegelförmige Reservoire 112 in entsprechende Vertiefungen eines Trägermaterials 113 eingebettet sind. Die Abgabeflächen 111 des Reservoirs 112 grenzen an eine Schutzschicht 114. Die geometrische Anordnung der Reservoire zueinander ist sehr variantenreich und richtet sich nach den Erfordernissen der Praxis.

Für die Kombination von zwei Reservoiren in einer Vorrichtung gibt Fig. 12 ein Beispiel, wobei der Längsschnitt einer Vorrichtung 120 dargestellt ist. Die Reservoire 122 sind wiederum in einem Trägermaterial 123 eingebettet. Das zentrale Reservoir 122 weist keine Querschnittsverkleinerungen auf und beeinhaltet einen ersten Wirkstoff, der nach der bekannten Freisetzungscharakteristik abgegeben wird. In dem zweiten Reservoir 122', das das erste Reservoir 122 ringförmig umgibt und dreieckigen Längsschnitt besitzt, ist ein zweiter Wirkstoff inkorporiert, der gesteuert abnehmend freigesetzt wird. Die Abgabeflächen 121, 121' stehen mit einer Haftklebeschicht 125 in Kontakt, die ihrerseits von einer Schutzschicht 124 abgedeckt ist. Den Querschnitt entlang der Linie I/I der Vorrichtung 120 gibt die Fig. 13 wieder. Die Anordnung der Reservoire 122, 122' im Trägermaterial 123 ist gut zu erkennen, wobei das mittige zylindrische Reservoir 122 des ersten Wirkstoffs ringförmig vom Reservoir 122' des zweiten Wirkstoffes umgeben ist.

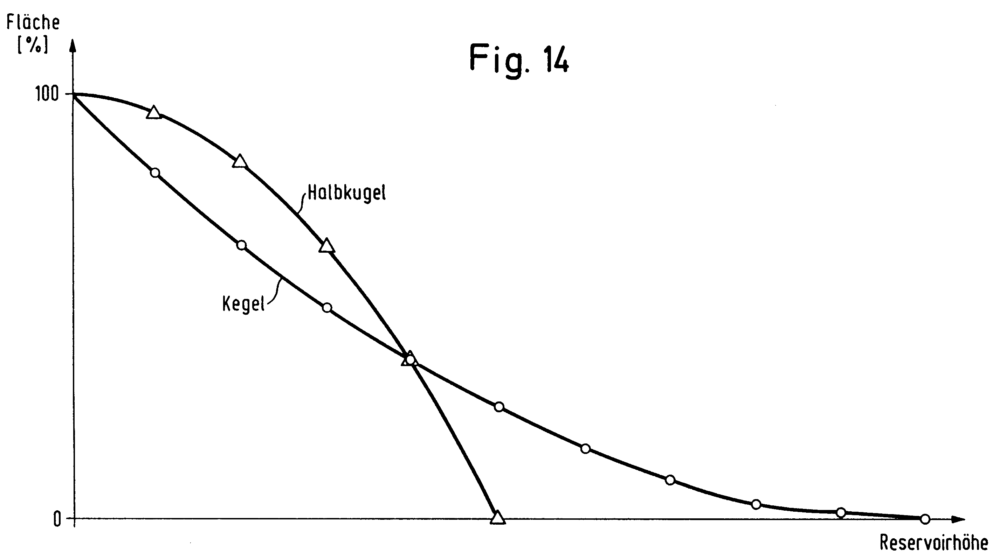

In Fig. 14 ist graphisch die Querschnittsverkleinerung bei kegel- und halbkugelförmigen Reservoiren dargestellt, wobei die Standfläche des Kegels bzw. die Halbkugel die Abgabefläche 91, 101 ist, wie in den Figuren 9 und 10 dargestellt. Es wird deutlich, daß beide geometrische Formen eine völlig verschiedene Abnahmecharakteristik besitzen. Beim Kegel ist ein langsames Abklingen zu verzeichnen, während bei der Halbkugel eine wesentlich höhere Anfangsabgabe festzustellen ist, die dann aber steil abfällt. In beiden Fällen ist die ursprüngliche Abgabefläche 91, 101 von gleicher Größe, der Kegelwinkel beträgt 53 Grad.

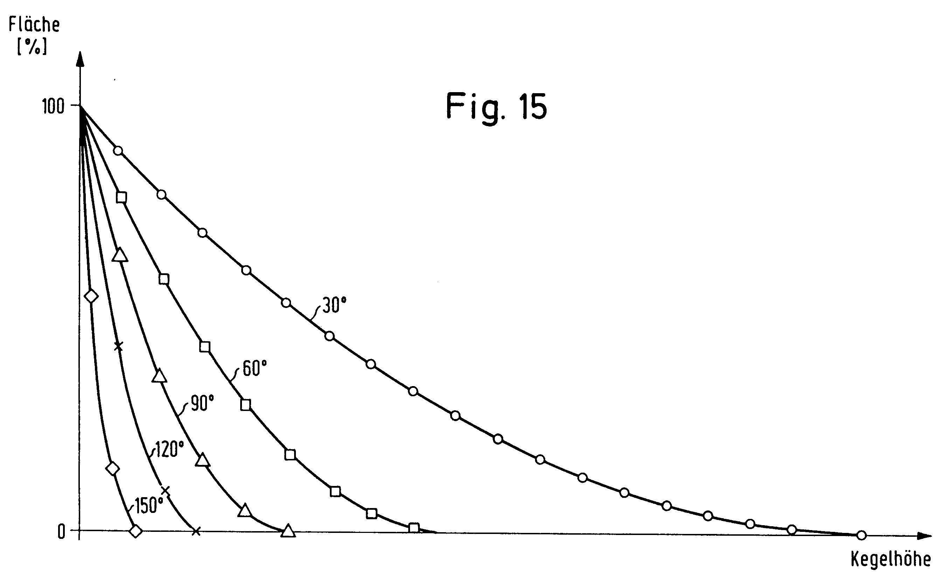

Die Veränderung der Abnahmecharakteristik des Querschnitts bei Kegeln gleicher Abgabefläche in Abhängigkeit vom Kegelwinkel geht aus Fig. 15 hervor. Es ist zu erkennen, daß durch die Wahl des Winkels fast jede beliebige ausschleichende Abnahmecharakteristik einstellbar ist.

### Beispiel

Aus 3 PVC-Quadern mit einer Kantenlänge von 4O x 4O mm und einer Höhe von 25 mm werden
a) ein Zylinder
b) ein Stufenzylinder (die untere Zylinderhälfte besitzt nur den halben Durchmesser der oberen Zylinderhälfte) und
c) ein Kegelstumpf
herausgebohrt, wobei alle Löcher in einer Tiefe von 2O mm eine Öffnung an der Quaderoberfläche von 314 mm² besitzen und die kleinere Fläche des Kegelstumpfes 14,1 mm² beträgt. In die so erhaltenen Löcher wurde aufgeschmolzenes Polyethylenglykol-6OOO (PEG-6OOO) bis ea. 3 mm unter den Rand eingefüllt. Nach Erstarren des PEG-6OOO in dem Prüfkörper wird weiteres aufgeschmolzenes PEG-6OOO nachgefüllt, bis sich eine etwa 2 mm hohe Erhebung auf dem Prüfkörper gebildet hat. Die nach Abkühlen überstehende Probesubstanz wird mit Hilfe eines Messers bis auf die Oberkante des Prüfkörpers abgetragen.

Die Freisetzung des PEG-6OOO in Wasser wird nach der "Paddle-over-disc"-Methode gemäß USP XX bestimmt. Der Prüfkörper wird alle 3O Minuten aus dem Bad entfernt und nach sorgfältiger Trocknung ausgewogen.

### Prüfbedingungen:

| | |
|---|---|
| Paddle-over-disc-Gerät: | SOTAX AT 6 (Sotax AG, Basel) |
| Freisetzungsnmedium: | 1OOO ml entmineralisiertes Wasser |
| Temperatur: | 35 Grad Celsius |
| Rührgeschwindigkeit: | 5O U/min |
| Rührhöhe über Prüfkörper: | 15 mm |

### Ergebnisse:

Es wurden folgende Freisetzungsraten (Flux(g/h)) gemessen:

### Zylindrischer Prüfkörper (Vergleichsversuch):

DieFreisetzungsgeschwindigkeit (Flux) aus einem zylindrischen Prüfkörper bleibt über ca 4 Stunden konstant bei etwa 1,8 g/h und Fällt sodann schnell auf Null ab.

### Stufenzylindrischer Prüfkörper:

Beim stufenzylindrischen Prüfkörper mit zwei Stufen wird zunächst bis etwa 2 h nach Versuchsbeginn eine gleichbleibende Freisetzungsgeschwindigkeit von etwa 1,8 g/h beobachtet, sodann findet ein dem Erreichen der Stufe entsprechender steiler Abfall der Freisetzungsgeschwindigkeit auf eine Freisetzungsgeschwindigkeit von O,3 g/h statt, die über etwa ein Stunde konstant bleibt und fällt sodann, nach Verbrauch der zweiten Stufe, auf Null ab.

### Kegelstumpf:

Beim kegelstumpfförmigen Reservoir Fällt die Freisetzungsgeschwindigkeit, gemessen als Flux in g/h gleichmäßig von etwa 1,8 g/h bei Versuchsbeginn auf Null innerhalb von vier Stunden, wobei die Abnahme hierbei kontinuierlich erfolgt, bzw. die Abnahmekurve des Flux, aufgetragen gegen die Versuchszeit, im wesentlichen eine Gerade ist.

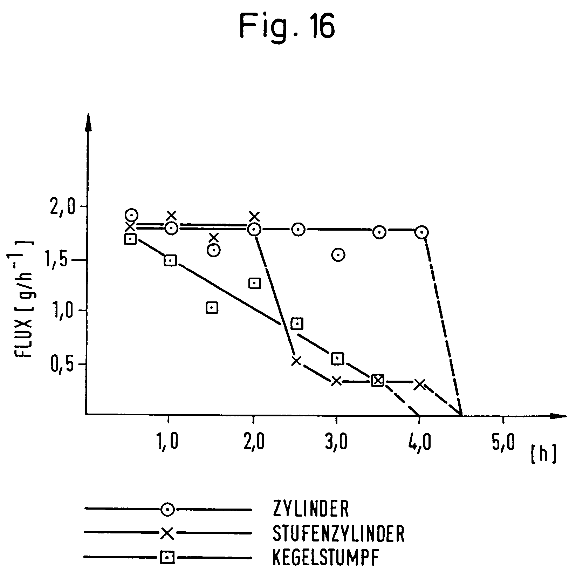

Die Ergebnisse des Versuches sind schematisch in Figur 16 dargestellt.

Die Figuren sind lediglich beispielhaft und sollen keineswegs einschränkend wirken.

## Patentansprüche

1. Verwendung einer Vorrichtung zur Abgabe von Wirkstoffen an einen Akzeptor zur über die Zeit der Anwendung gesteuert abnehmenden Abgabe von Wirkstoffen, mit mindestens einem Wirkstoffreservoir mit einer Abgabefläche, die mit dem Akzeptor in Kontakt steht, wobei mindestens eine zur Abgabefläche parallele Querschnittsfläche des Reservoirs kleiner ist als die Abgabefläche und keine zur Abgabefläche parallele Querschnittsfläche des Reservoirs größer ist als die Abgabefläche und durch die geometrische Form des Reservoirs die Abgabecharakteristik bestimmt ist.

2. Verwendung nach Anspruch 1, dadurch gekennezeichnet, daß die Querschnittsverkleinerung kontinuierlich oder diskontinuierlich, linear oder nicht linear ist.

3. Verwendung nach Anspruch 2, dadurch gekennzeichnet, daß das Reservoir die Form eines Kegels oder Kegelstumpfes, einer Pyramide oder eines Pyramidenstumpfes, eines Tetreders oder eines Tetrederstumpfes, einer Halbkugel, eines Kugelsegments oder einer Kugelschicht aufweist.

4. Verwendung nach Anspruch 1 oder 2, dadurch gekennzeichnet, daß die Querschnittsflächeverkleinerung nur in einem Teil des Reservoirs ausgebildet ist.

5. Verwendung nach Anspruch 1 oder 2, dadurch gekennzeichnet, daß das Reservoir keine Symmetrielemente aufweist.

6. Verwendung nach einem der vorgehenden Ansprüche, dadurch gekennzeichnet, daß das Reservoir eine Abgabefläche besitzt, die in einer Richtung eine maximale Ausdehnung aufweist, die bis zum 100-fachen der Ausdehnung in der dazu senkrechten Richtung beträgt.

7. Verwendung nach einer Vorrichtung zur Abgabe von Wirkstoffen an einen Akzeptor, wobei alle Oberflächen des Reservoirs, ausgenommen die Abgabefläche mit mindestens einer für die Reservoirbestandteile undurchlässigen Schicht versehen sind.

8. Verwendung nach irgend einen der vorangehenden Ansprüche, dadurch gekennzeichnet, daß das Reservoir mit mindestens einer Fläche, ausgenommen die Abgabefläche, an mindestens einer Innenwand einer für die Aufnahme mindestens eines Teils des Reservoirs eingerichteten Vertiefung in einem Trägermaterial fixiert ist.

9. Verwendung nach einem der vorangehenden Ansprüche, dadurch gekennzeichnet, daß die Abgabefläche des Reservoirs vor dessen Anwendung durch ein mindestens einschichtiges, wieder ablösbares und für die Bestandteile des Reservoirs undurchlässiges Flächengebilde geschützt ist.

10. Verwendung nach einem der vorangehenden Ansprüche, dadurch gekennzeichnet, daß die Vorrichtung am Ort der Anwendung fixierbar ist.

11. Verwendung nach einem der vorangehenden Ansprüche, dadurch gekennzeichnet, daß die Vorrichtung durch haftklebende Bereiche fixierbar ist.

12. Verwendung nach einem der vorangehenden Ansprüche, dadurch gekennzeichnet, daß die Vorrichtung mindestens ein Wirkstoff, der im technischen, human- und veterinärmedizinischen, kosmetischen und Schädlingsbekämpfungs-Bereich wirksam ist, aufweist.

13. Verwendung einer Vorrichtung nach einem der vorangehenden Anspürche als transdermales therapeutisches System.

## Claims

1. Use of a device for the release of active substances to an acceptor for the release of active substances which decreases in a controlled manner over the period of use, having at least one active substance reservoir provided with a release area, which is in contact with the acceptor, at least one cross-sectional area of the reservoir parallel to the release area being smaller than the release area, and no cross-sectional area of the reservoir parallel to the release area being greater than the release area and the release characteristic being determined by the geometrical configuration of the reservoir.

2. Use according to claim 1, characterised in that the cross-sectional reduction is continuous or discontinuous, linear or non-linear.

3. Use according to claim 2, characterised in that the reservoir has the configuration of a cone or truncated cone, a pyramid or a truncated pyramid, a tetrahedron or a truncated tetrahedron, a hemisphere, a segment of a sphere or a layer of a sphere.

4. Use according to claim 1 or 2, characterised in that the reduction in cross-sectional area is only provided in one portion of the reservoir.

5. Use according to claim 1 or 2, characterised in that the reservoir has no elements of symmetry.

6. Use according to one of the preceding claims, characterised in that the reservoir comprises a release area which has a maximum expansion in one direction which amounts to 100 times the expansion in the direction perpendicular thereto.

7. Use of a device for the release of active substances to an acceptor, all of the surfaces of the reservoir, except the release area, being provided with at least one layer which is impermeable to the constituent ingredients of the reservoir.

8. Use according to any one of the preceding claims, characterised in that the reservoir is secured by at least one area, except the release area, to at least one internal wall of a recess in a carrier material, which recess is provided to accommodate at least one portion of the reservoir.

9. Use according to one of the preceding claims, characterised in that the release area of the reservoir is protected from being used by means of a surface structure which is at least one-layered, is detachable again and is impermeable to the constituent ingredients of the reservoir.

10. Use according to one of the preceding claims, characterised in that the device is securable at the place of use.

11. Use according to one of the preceding claims, characterised in that the device is securable by means of adhesive regions.

12. Use according to one of the preceding claims, characterised in that the device has at least one active substance, which is effective in the technical field, in the field of human and veterinary medicine, in the field of cosmetics and in the field of pest control.

13. Use of a device according to one of the preceding claims as a transdermal, therapeutic system.

## Revendications

1. Utilisation d'un dispositif de libération de matières actives sur un milieu récepteur afin de permettre la libération de matières actives à décroissance commandée sur le temps d'application, ledit dispositif comprenant au moins un réservoir de matière active présentant une surface de libération, qui vient en contact avec le milieu récepteur, au moins une surface en coupe transversale du réservoir parallèle à la surface de libération étant plus petite que la surface de libération, aucune surface en coupe transversale du réservoir parallèle à la surface de libération n'étant plus grande que la surface de libération et la caractéristique de libération étant déterminée par la forme géométrique du réservoir.

2. Utilisation selon la revendication 1, caractérisée en ce que la réduction en coupe transversale est continue ou discontinue, linéaire ou non linéaire.

3. Utilisation selon la revendication 2, caractérisée en ce que le réservoir a la forme d'un cône ou d'un tronc de cône, d'une pyramide ou d'un tronc de pyramide, d'un tétraèdre ou d'un tronc de tétraèdre, d'une demi-sphère, d'un segment de sphère ou d'une couche sphérique.

4. Utilisation selon la revendication 1 ou 2, caractérisée en ce que la réduction de surface en coupe transversale ne se présente que dans une partie du réservoir.

5. Utilisation selon la revendication 1 ou 2, caractérisée en ce que le réservoir ne présente pas d'éléments de symétrie.

6. Utilisation selon l'une quelconque des revendications précédentes, caractérisée en ce que le réservoir présente une surface de libération qui comporte, dans une direction, une extension maximum qui atteint jusqu'à 100 fois l'extension dans la direction perpendiculaire à la première direction.

7. Utilisation d'un dispositif de libération de matières actives sur un milieu récepteur, toutes les surfaces du réservoir, à l'exclusion de la surface de libération, étant pourvues d'au moins une couche qui ne laisse pas passer les composants du réservoir.

8. Utilisation selon l'une quelconque des revendications précédentes, caractérisée en ce que le réservoir est fixé par au moins une surface, à l'exclusion de la surface de libération, sur au moins une paroi interne d'un renfoncement aménagé dans une matière de support pour la réception d'au moins une partie du réservoir.

9. Utilisation selon l'une quelconque des revendications précédentes, caractérisée en ce que la surface de libération du réservoir est protégée, avant son application, par un élément superficiel au moins monocouche, ré-enlevable et qui ne laisse pas passer les composants du réservoir.

10. Utilisation selon l'une quelconque des revendications précédentes, caractérisée en ce que le dispositif peut être fixé au point d'application.

11. Utilisation selon l'une quelconque des revendications précédentes, caractérisée en ce que le dispositif peut être fixé par des zones autocollantes.

12. Utilisation selon l'une quelconque des revendications précédentes, caractérisée en ce que le dispositif comporte au moins une matière active, qui s'avère efficace dans le domaine technique, en médecine humaine et vétérinaire, dans le secteur des cosmétiques et dans celui de la lutte antiparasites.

13. Utilisation d'un dispositif selon l'une quelconque des revendications précédentes comme système thérapeutique transdermique.
